# EUROPÄISCHE PATENTANMELDUNG

(11) **EP 1 845 369 A1**
(43) Veröffentlichungstag der Anmeldung: **17.10.2007**
(21) Anmeldenummer: 07102724.7
(22) Anmeldetag: 20.02.2007
(51) Int. Cl.: G01N 27/414

(54) **Gassensor**

(30) Priorität: 12.04.2006 DE 102006017172
(71) Anmelder: ROBERT BOSCH GMBH, 70442 Stuttgart (DE)
(72) Erfinder: Cramer, Berndt, 71229, Leonberg (DE); Schumann, Bernd, 71277, Rutesheim (DE); Thiemann-Handler, Sabine, 70435, Stuttgart (DE); Ziegler, Joerg, 71277, Rutesheim (DE); Roessler, Mario, 70176, Stuttgart (DE)

(57) **Zusammenfassung**

Die vorliegende Erfindung betrifft einen Sensor zur Bestimmung einer Gaskomponente beziehungsweise deren Konzentration in einem Messgas und/oder zur Bestimmung einer physikalischen Größe des Messgases wie zum Beispiel Temperatur, Gasdruck, Partikelkonzentration oder dergleichen mit einem keramischen oder metallkeramischen Trägerkörper (2). Sie zeichnet sich dadurch aus, dass ein Detektionselement (3) des Sensors (1) als Halbleitersensor (3) aufgebaut ist.

## Beschreibung

Die vorliegende Erfindung betrifft einen Sensor zur Bestimmung einer Gaskomponente beziehungsweise deren Konzentration in einem Messgas und/oder zur Bestimmung einer physikalischen Größe des Messgases oder einer Partikelkonzentration nach dem Oberbegriff des Anspruchs 1.

### Stand der Technik

Zur Ermittlung einer Gaskomponente beziehungsweise deren Konzentration in einem Messgas und/oder zur Bestimmung einer physikalischen Größe des Messgases wie Temperatur, Gasdruck, Partikelkonzentration oder dergleichen sind Sensoren bekannt, die auf unterschiedlichen Technologien aufbauen.

Beispielsweise kann mittels sogenannter Lambdasonden die Bestimmung des Gleichgewichtssauerstoffgehaltes in Automobilabgasen ermittelt werden. Hierbei wird mittels eines in der Regel in Planartechnik aufgebauten elektrochemischen, festkörperelektrolytischen Sensors, ein vom Gleichgewichtssauerstoffgehalt (Gleichgewichtssauerstoff-Potentialdruck) im Abgas abhängiges Steuersignal erzeugt. Die Betriebstemperatur im Inneren solcher Lambdasonden liegt im Bereich von ca. 750 °C und darüber. Selbst in Randbereichen solcher Lambdasonden herrschen immer noch Temperaturen im Bereich von deutlich über 300 °C.

Diese vergleichsweise hohen Betriebstemperaturen, sowie auch zum Teil sehr aggressive Einsatzbedingungen über den Lebenszyklus eines solchen Sensors, wie Säurekontakt und dgl., die insbesondere in Abgassträngen auftreten, schränken die Auswahl ausreichend robuster Stoffe zur Herstellung solcher Sensoren und damit die Bereitstellung unterschiedlicher Sensorfunktionalitäten an einem solchen Sensor stark ein.

### Aufgabe und Vorteile der Erfindung

Der vorliegenden Erfindung liegt daher die Aufgabe zugrunde, einen Sensor entsprechend der einleitend beschriebenen Gattung zu verbessern.

Diese Aufgabe wird durch die Merkmale der Ansprüche 1 und 14 gelöst. In den Unteransprüchen sind vorteilhafte und zweckmäßige Weiterbildungen der Erfindung angegeben.

Dementsprechend betrifft die vorliegende Erfindung einen Sensor zur Bestimmung einer Gaskomponente beziehungsweise deren Konzentration in einem Messgas und/oder zur Bestimmung einer physikalischen Größe des Messgases wie Temperatur, Gasdruck oder dergleichen oder einer Partikelkonzentration, mit einem keramischen oder metall-keramischen Trägerkörper. Dieser Sensor zeichnet sich erfindungsgemäß dadurch aus, dass ein Detektionselement des Sensors als Halbleitersensor ausgebildet ist. Insbesondere kann es sich bei einem solchen Halbleitersensor um einen entsprechende Strukturen aufweisenden Hochtemperaturhalbleiter handeln, der beispielsweise aus SiC, GaN oder GaAlN aufgebaut ist. Diese Halbleiter sind für den Einsatz in Temperaturbereichen > 300 °C geeignet und können somit auf einen entsprechend robusten Träger aufgebracht und einem Messgas, insbesondere einem Abgas einer Verbrennungsvorrichtung zur Bestimmung einer oder auch mehrerer der oben angeführten Messgrößen aufgesetzt werden.

Eine mögliche Struktur des Halbleitersensors zur Bestimmung einer Gaskomponente könnte beispielsweise ein Feldeffekttransistor sein, der abhängig von der zu bestimmenden Größe des Messgases ein Signal verändert. In einer Ausführungsform könnte beispielsweise ein für polare Moleküle sensitives Gate an dem Feldeffekttransistor vorgesehen sein.

In einer weiteren Ausführungsform könnte beispielsweise ein sich abhängig von der Konzentration eines bestimmten Gases verändernder elektrischer Widerstand an dem Halbleitersensor vorgesehen sein. Solche Sensoren werden auch resistive Sensoren genannt. Eine mögliche Stoffkombination zum Aufbau eines solchen Sensors wäre beispielsweise Strontium-Titanat. Damit ist die Ausbildung von O²⁻-Fehlstellen möglich, so dass bei hohem Sauerstoffgehalt im Messgas diese Fehlstellen abnehmen, so dass die elektrische Leitfähigkeit abhängig vom Sauerstoffpartialdruck sinkt beziehungsweise der elektrische Widerstand ansteigt.

In einer vorteilhaften Weiterbildung kann ein solcher Sensor auch ein Heizelement umfassen, so dass damit einerseits eine bestimmte Betriebstemperatur eingestellt werden kann und andererseits auch die Möglichkeit besteht, den Sensor so weit zu erhitzen, dass gegebenenfalls an ihm anhaftende Niederschläge verdampft und/oder verbrannt werden können. Dadurch kann der Sensor aktiv vor Partikelablagerungen beziehungsweise Kondensatbildung durch Niederschlag geschützt werden, was einerseits die Signalstabilität über Betriebszeit des Sensors hinweg deutlich verbessert, und andererseits korrosiven Einwirkungen durch aggressive Stoffgemische an der Oberfläche des Sensor zumindest zum Teil entgegenwirkt.

Eine weitere Maßnahme gegen schädigende Einwirkungen auf den Sensor, zum Beispiel hervorgerufen durch Materialspannungen aufgrund der hohen Temperaturschwankungen zwischen Starttemperatur und Betriebstemperatur des Sensors, kann die zur Verfügungstellung von Mitteln zur Kompensation unterschiedlicher Materialausdehnungen des Keramikkörpers und des Halbleitersensors sein. Solche Mittel können beispielsweise in ein Anschlusselement für den Halbleiter integriert sein. So können beispielsweise über die Längserstreckung des Anschlusselementes unterschiedliche Materialausdehnungen an dessen beiden Anschlussenden flexibel abgefedert werden. Eine mögliche Ausführungsform könnte beispielsweise ein Bond-Draht sein, mit dem der Halbleiter kontaktiert und am Trägerkörper befestigt ist.

Eine weitere Schutzmöglichkeit des Sensors insbesondere des Halbleitersensors kann durch eine flüssigkeits- und/oder gasdichte Schutzschicht realisiert werden, die zumindest einen Teil der Sensoroberfläche, insbesondere den Bereich, auf welchem der Halbleitersensor angeordnet ist, bedeckt. Als besonders vorteilhaft wird es angesehen, wenn diese oder auch eine andere Schicht auch noch eine elektrisch isolierende Eigenschaft aufweist, so dass insbesondere durch Feuchtigkeit keine Messsignalstörungen oder gegebenenfalls Schädigungen des Sensors z. B. durch Reduzierung elektrischer Widerstände bis hin zum Kurzschluss auftreten können.

In einer besonders bevorzugten Ausführungsform ist der Sensor mit einer Temperaturisolationsschicht versehen, welche insbesondere vorteilhaft zwischen dem Heizelement und dem Halbleitersensor ausgebildet ist. Dadurch können sehr effektiv sogenannte "Hot Spots", also Bereiche mit vergleichsweise stark überhöhter Temperatur gegenüber anderen Bereichen des Sensors vermieden beziehungsweise gegenüber dem Halbleitersensor unwirksam gemacht werden. Weiterhin ist damit eine zusätzlich gleichmäßigere Temperaturverteilung im Inneren des Sensors und insbesondere gegenüber dem auf dem Keramikelement aufgesetzten Halbleitersensor möglich, so dass auch hierdurch eine weitere Reduzierung von temperaturbedingten Materialspannungen bewirkt werden kann.

Der Vorteil einer solchen Temperaturisolationsschicht kommt besonders dann zum Tragen, wenn, wie in einer weiteren besonders bevorzugten Ausführungsform, der Sensor zusätzlich mit einem weiteren Detektionselement oder auch Sensor zur Bestimmung einer weiteren Gaskomponente beziehungsweise deren Konzentration in dem Messgas und/oder zur Bestimmung einer weiteren physikalischen Größe des Messgases, wie zum Beispiel Temperatur, Gasdruck, Partikelkonzentration oder dergleichen versehen ist, welche gegebenenfalls auf eine im Vergleich zu der bisher beschriebenen Ausführungsform höhere Betriebstemperatur aufgeheizt werden muss. Dies kann beispielsweise dann der Fall sein, wenn dieser zusätzliche Sensor auf der Basis von Zirkonoxid arbeitet, welches eine Betriebstemperatur von etwa 750 °C benötigt, um z. B. eine ausreichende Ionenleitung zur Bestimmung des Gleichgewichtssauerstoffgehaltes in einem Messgas zu ermöglichen. In einer solchen Ausführungsform kann beispielsweise ein Heizelement sowohl den Ionenleiter auf Betriebstemperatur in Größenordnungen von 750 °C und zum Teil auch deutlich darüber hinaus bringen und gleichzeitig auch den Halbleitersensor möglichst gleichmäßig temperieren und seine Oberfläche vor verunreinigenden Ablagerungen schützen.

Durch die Kombination dieses weiteren Detektionselements bzw. Sensors, das bzw. der beispielsweise eine Lambdasonde, ein NOₓ-Sensor, ein Drucksensor oder irgendein anderer eine bestimmte physikalische Größe oder eine Partikelkonzentration des Messgases detektierender Sensor, mit einem hochtemperaturbeständigen Halbleitersensor sein kann, kann somit jede beliebige Kombination an Detektierungseinheiten mit einem erfindungsgemäßen Sensor auf engstem Raum realisiert werden. Besonders vorteilhaft wirkt sich diese Möglichkeit bei der Erfassung von Gaskonzentrationen aus, bei denen das Messgas das Abgas einer Verbrennungsanlage, insbesondere eines Verbrennungsmotors ist. Gerade im Fahrzeugbau besteht permanent das Bestreben, die Zusammensetzung des Abgases immer besser bestimmen zu können, wozu immer mehr Sensoren eingesetzt werden müssen, deren Gesamtanzahl durch den erfindungsgemäßen Sensor nun wiederum reduziert werden kann, beziehungsweise wodurch eine größere Anzahl von zu detektierenden Parametern, wenn erforderlich in einem Gehäuse, abgedeckt werden kann.

Eine für den Anschluss an den Sensor vorgesehene Kontrolleinheit kann im Weiteren zur Auswertung und/oder Aufbereitung der verschiedenen Signale des Sensors, also sowohl des Halbleitersensors als auch des zusätzlichen Sensors vorgesehen sein. Insbesondere durch Hinterlegung bestimmter Algorhythmen und/oder Parameterscharen können für den jeweiligen Anwendungsfall gezielt auf den jeweils vorgesehenen Betriebstemperaturbereich abgestellte Beeinflussungen und/oder Korrekturen der Sensorsignale vorgenommen werden.

Im Weiteren betrifft die vorliegende Erfindung auch ein Verfahren zur Bestimmung einer Gaskomponente bzw. deren Konzentration in einem Messgas und/oder zur Bestimmung einer physikalischen Größe des Messgases, wie z. B. Temperatur, Gasdruck oder dergleichen, oder einer Partikelkonzentration, bei dem ein Sensor mit einem keramischen oder metallkeramischen Trägerkörper verwendet wird, und das sich dadurch auszeichnet, dass als Detektionselement des Sensors ein Halbleitersensor verwendet wird.

### Ausführungsbeispiel

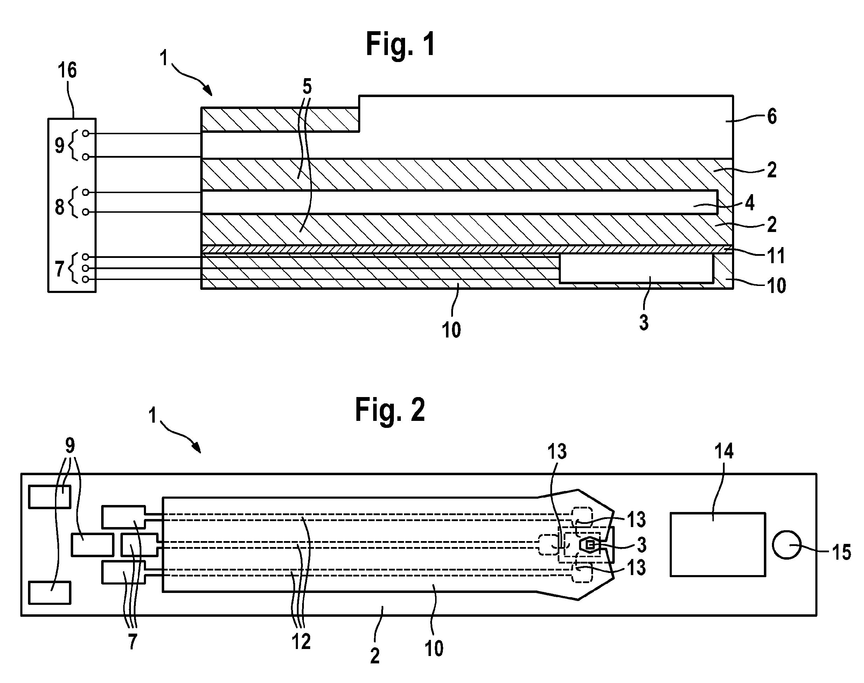

Die Erfindung wird an Hand der Zeichnungen und der nachfolgenden, darauf bezugnehmenden Beschreibung näher erläutert. Es zeigen
- Figur 1: einen Sensor zur Bestimmung einer Gaskomponente beziehungsweise deren Konzentration in einem Messgas und/oder zur Bestimmung einer physikalischen Größe des Messgases in einer schematischen Schnittdarstellung und
- Figur 2: den Sensor nach Figur 1 in einer schematischen Draufsicht.
Im Detail zeigt die schematische Schnittdarstellung der Figur 1 einen Sensor 1 mit einem keramischen oder metallkeramischen Trägerkörper 2, auf dem erfindungsgemäß ein Detektionselement 3 in der Form eines Halbleitersensors 3 angeordnet ist. Der Halbleitersensor ist auf einem hochtemperaturbeständigen Halbleiterträger aufgebaut und kann beispielsweise mit einem Feldeffekttransistor ausgestattet sein. Zur Verstärkung des Messsignals können auch mehrere Feldeffekttransistoren in dem hochtemperaturbeständigen Halbleitersensor untergebracht sein. Diese haben dann vorzugsweise alle den gleichen Aufbau beziehungsweise die gleiche Sensoreigenschaft.

Denkbar ist aber auch die Anordnung mehrerer Feldeffekttransistoren mit unterschiedlichen Sensoreigenschaften. Dann können mit einem Halbleitersensor verschiedene Parameter des Messgases bestimmt werden. Eine mögliche Ausführungsform eines Feldeffekttransistor könnte z. B. mit einer für polare Moleküle sensitiven Schicht als Gate realisiert werden, so dass durch Anlagerungen von Molekülen an dieser Schicht eine entsprechende Veränderung der Leitfähigkeit des Transistorkanals hervorgerufen wird. Durch Beaufschlagung mit einer elektrischen Spannung bzw. mit einem elektrischen Strom kann diese Änderung als Sensorsignal abgegriffen werden.

Denkbar ist aber auch, dass der Halbleitersensor einen sich abhängig von der Konzentration eines bestimmten Gases verändernden elektrischen Widerstand aufweist.

Zur Einstellung der Betriebstemperatur des Sensors 1 ist im Weiteren eine Heizung 4 vorgesehen. Diese kann beispielsweise, elektrisch isoliert, im Inneren des Trägerkörpers 2 untergebracht sein. Der Trägerkörper 2 kann wiederum eine weitere Funktion innehaben, zum Beispiel die eines Ionenleiters 5 für einen weiteren, im Sensor 1 untergebrachten Sensor 6, zum Beispiel in der Form einer Lambdasonde. Der Sensor 6 kann aber durchaus auch ein Sensor zur Erfassung einer physikalischen Größe eines Messgases sein, wie zum Beispiel zur Erfassung der Temperatur, eines Gasdrucks, einer Partikelkonzentration oder auch zur Detektierung einer Kombination mehrerer Parameter eines zu detektierenden Messgases.

Die Positionen 7 bis 9 stellen beispielhaft elektrische Anschlüsse der verschiedenen Elemente des Sensors 1 dar.

Zum Schutz der Oberfläche des Sensors 1, insbesondere zum Schutz des Halbleitersensors 3 ist eine Schicht 10 vorgesehen, die neben einer flüssigkeitsdichten Eigenschaft vorzugsweise auch noch elektrisch isolierend ist. Eine Temperaturisolationsschicht der Heizung 4 und des hochtemperaturbeständigen Halbleitersensors 3 ist als Schicht 11 dargestellt.

Die Figur 2 zeigt eine schematische Draufsicht auf einen solchen Sensor 1, bei dem auf dem Trägerkörper 2 neben den Kontakten 7 und 9 Leiterbahnen als beispielhafte Anschlussverbindungen für den hochtemperaturbeständigen Halbleitersensor 3 unter der Schicht 10 zu erkennen sind, welche ihrerseits wiederum beispielsweise aus einem Glas hergestellt sein kann. Die Glasart kann zum Beispiel ein Erdalkalialumosilikatglas mit Zirkonium oder Titanzusatz sein.

Die Anschlüsse 13 des Halbleitersensors 3 sind hier beispielhaft als Bond-Drähte ausgeführt, und dienen gleichzeitig als Mittel 13 zur Kompensation unterschiedlicher Materialausdehnungen des Keramikkörpers 2 und des Halbleitersensors 3, zum Beispiel aufgrund der großen Temperaturschwankungen zwischen Start- und Betriebstemperatur des Sensors 1.

Im Weiteren ist unter der Nummer 14 eine sogenannte "äußere Pumpelektrode" beispielhaft für einen Sauerstoffsensor, zum Beispiel einer Lambdasonde, dargestellt, der beispielsweise durch Sensor 6 realisiert sein kann. Die Öffnung 15 kann in einem solchen Aufbau beispielsweise zur Versorgung des Sensors mit dem Messgas dienen. In einem anderen Ausführungsbeispiel könnte hierdurch auch die Erfassung eines Gasdrucks oder anderen physikalischen Größe des Gases vorgesehen sein.

Die kombinierte Unterbringung des hochtemperaturbeständigen Halbleitersensors 3 und eines weiteren konventionellen Sensors 6 ermöglicht somit unterschiedlich aufgebaute Ausführungsformen zur Erfassung verschiedenster Kombination von Parametern eines zu bestimmenden Messgases, welcher auf einem Keramik- oder einem Metall-Keramikgrundkörper aufgebaut ist.

Zum Betrieb beziehungsweise zur Ansteuerung dieses Sensors dient die schematisch dargestellte Kontrolleinheit 16.

## Patentansprüche

1. Sensor (1) zur Bestimmung einer Gaskomponente bzw. deren Konzentration in einem Messgas und/oder zur Bestimmung einer physikalischen Größe des Messgases, wie z.B. Temperatur, Gasdruck oder dgl. oder einer Partikelkonzentration, mit einem keramischen oder metall-keramischen Trägerkörper (2), **dadurch gekennzeichnet, dass** ein Detektionselement (3) des Sensors (1) als Halbleitersensor (3) aufgebaut ist.

2. Sensor nach Anspruch 1, **dadurch gekennzeichnet, dass** der Halbleitersensor (3) einen Feldeffekttransistor umfasst.

3. Sensor nach Anspruch 1 oder 2, **dadurch gekennzeichnet, dass** der Feldeffekttransistor ein für polare Moleküle sensitives Gate aufweist.

4. Sensor nach einem der vorangehenden Ansprüche, **dadurch gekennzeichnet, dass** der Halbleitersensor (3) einen sich abhängig von der Konzentration eines bestimmten Gases verändernden elektrischen Widerstand aufweist.

5. Sensor nach einem der vorangehenden Ansprüche, **dadurch gekennzeichnet, dass** ein Heizelement (4) vorgesehen ist.

6. Sensor nach einem der vorangehenden Ansprüche, **dadurch gekennzeichnet, dass** Mittel (13) zur Kompensation unterschiedlicher Materialausdehnungen des Keramikkörpers (2) und des Halbleitersensors (3) vorgesehen sind.

7. Sensor nach einem der vorangehenden Ansprüche, **dadurch gekennzeichnet, dass** die Mittel zur Kompensation unterschiedlicher Materialausdehnungen als elektrisches Anschlusselement (13) für den Halbleitersensor (3) ausgebildet sind.

8. Sensor nach einem der vorangehenden Ansprüche, **dadurch gekennzeichnet, dass** eine flüssigkeitsdichte Schutzschicht (10) für zumindest einen Teil der Sensoroberfläche vorgesehen ist.

9. Sensor nach einem der vorangehenden Ansprüche, **dadurch gekennzeichnet, dass** eine elektrisch isolierende Schutzschicht (10) für zumindest einen Teil der Sensoroberfläche vorgesehen ist.

10. Sensor nach einem der vorangehenden Ansprüche, **dadurch gekennzeichnet, dass** eine Temperaturisolationsschicht (11) vorgesehen ist.

11. Sensor nach einem der vorangehenden Ansprüche, **dadurch gekennzeichnet, dass** ein weiteres Detektionselement (6) zur Bestimmung einer weiteren Gaskomponente bzw. deren Konzentration in dem Messgas und/oder zur Bestimmung einer weiteren physikalischen Größe des Messgases wie z. B. Temperatur, Gasdruck Partikelkonzentration oder dgl. vorgesehen ist.

12. Sensor nach einem der vorangehenden Ansprüche, **dadurch gekennzeichnet, dass** das weitere Detektionselement (6) einen elektrochemischen Festkörperelektrolytsensor (5) umfasst.

13. Sensor nach einem der vorangehenden Ansprüche, **dadurch gekennzeichnet, dass** eine Kontrolleinheit (16) für den Anschluss an den Sensor (1) vorgesehen ist.

14. Verfahren zur Bestimmung einer Gaskomponente bzw. deren Konzentration in einem Messgas und/oder zur Bestimmung einer physikalischen Größe des Messgases, wie z. B. Temperatur, Gasdruck oder dgl. oder einer Partikelkonzentration, wobei ein Sensor (1) mit einem keramischen oder metallkeramischen Trägerkörper (2) verwendet wird, **dadurch gekennzeichnet, dass** als Detektionselement (3) des Sensors (1) ein Halbleitersensor (3) verwendet wird.
